Europäisches Patentamt

European Patent Office

Office européen des brevets

(11) Numéro de publication: **0 441 451 B1**

(12) **FASCICULE DE BREVET EUROPEEN**

(45) Date de publication de fascicule du brevet: **03.05.95** (51) Int. Cl.⁶: **A61B 8/06**

(21) Numéro de dépôt: **91200247.4**

(22) Date de dépôt: **06.02.91**

(54) **Dispositif de mesure de la vitesse d'organes en mouvement et d'écoulements sanguins par échographie ultrasonore, à vitesse limite de mesure élevée.**

(30) Priorité: **09.02.90 FR 9001530**

(43) Date de publication de la demande:
**14.08.91 Bulletin 91/33**

(45) Mention de la délivrance du brevet:
**03.05.95 Bulletin 95/18**

(84) Etats contractants désignés:
**DE FR GB**

(56) Documents cités:
**EP-A- 0 159 013**
**EP-A- 0 338 618**
**FR-A- 2 590 790**
**US-A- 4 534 357**

(73) Titulaire: **LABORATOIRES D'ELECTRONIOUE PHILIPS**
**22, Avenue Descartes**
**F-94450 Limeil-Brévannes (FR)**
(84) Etats contractants désignés:
**FR**

(73) Titulaire: **Philips Electronics N.V.**
**Groenewoudseweg 1**
**NL-5621 BA Eindhoven (NL)**
(84) Etats contractants désignés:
**DE GB**

(72) Inventeur: **Bonnefous, Odile, Société Civile S.P.I.D.**
**156, Boulevard Haussmann**
**F-75008 Paris (FR)**
Inventeur: **Mangotte, Frédéric, Société Civile S.P.I.D.**
**156, Boulevard Haussmann**
**F-75008 Paris (FR)**

(74) Mandataire: **Pyronnet, Jacques et al**
**Société Civile S.P.I.D.**
**156, Boulevard Haussmann**
**F-75008 Paris (FR)**

## EP 0 441 451 B1

**Description**

La présente invention concerne un dispositif de mesure de la vitesse d'organes en mouvement et d'écoulements sanguins par échographie ultrasonore utilisant une onde acoustique de fréquence $f_o$, et comportant une première voie de traitement du signal échographique reçu, comprenant un premier circuit d'intercorrélation délivrant $2l+1$ valeurs échantillonnées de la fonction de corrélation de deux signaux échographiques $S_n(t)$ et $S_{n+1}(t)$ successifs.

L'invention trouve une application particulièrement avantageuse dans le domaine de l'exploration échographique d'organes en mouvement, tels que les parois du coeur, et d'écoulements sanguins dans les vaisseaux.

Le problème technique général à résoudre par tout dispositif de mesure de la vitesse d'organes en mouvement et d'écoulements sanguins est d'obtenir une estimation aussi exacte que possible de la vitesse axiale du mouvement étudié afin de réaliser, à l'aide de dispositifs de visualisation, des images précises des organes et des écoulements sanguins soumis à une exploration échographique ultrasonore.

Depuis plusieurs années, diverses solutions à ce problème technique ont été proposées. En ce sens, la demande de brevet européen n° 0 225 667 décrit un dispositif de mesure de la vitesse d'organes en mouvement et d'écoulements sanguins conforme au préambule qui utilise le fait que les signaux ultrasonores successifs rétrodiffusés par une cible en mouvement, lorsque l'émission est récurrente à la période de récurrence T, sont reliés par l'équation suivante :

$$S_{n+1}(t) = S_n(t-\tau) \qquad (1)$$

Ceci signifie que le signal $n+1$ est la réplique du signal $n$ précédent à un décalage temporel $\tau$ près. Ce dernier représente le temps supplémentaire nécessaire à l'onde ultrasonore pour parcourir le trajet transducteur-cible-transducteur, d'un tir à l'autre. Autrement dit :

$$\tau = 2VT/C$$

où V est la vitesse de la cible et C la vitesse du son. On voit qu'une mesure de $\tau$ permet de mesurer la vitesse V cherchée.

La fonction d'intercorrélation entre $S_n(t)$ et $S_{n+1}(t)$ définie par :

$$C_{n,n+1}(to,u) = \int_{to}^{to+W} S_{n+1}(t+u)S_n(t)dt$$

vérifie :

$$C_{n,n+1}(to,u) = C_{nn}(to,u-\tau)$$

Le temps to est lié à la profondeur d'exploration z par $to = 2z/C$ et W est la fenêtre d'intégration.

La fonction $C_{nn}(to,u)$ est une fonction d'autocorrélation, et, de ce fait, est maximale pour $u = o$. Ainsi, une mesure du décalage temporel $\tau$, et donc de la vitesse V, peut se faire en cherchant pour quel paramètre u la fonction $C_{n,n+1}(to,u)$ est maximale. A cet effet, la fonction d'intercorrélation est échantillonnée, à un pas d'échantillonnage $\Delta t$, entre $u_{min} = -l\Delta t$ et $u_{max} = l\Delta t$ par pas de 1 de façon à obtenir $2l+1$ valeurs de fonctions de corrélation. La valeur maximum de ces $2l+1$ valeurs correspondant à $u = uo$ permet de mesurer $\tau$ en utilisant l'égalité $\tau = uo$.

Afin de s'affranchir des erreurs inhérentes à l'échantillonnage dans la détermination du maximum de la fonction de corrélation, il est possible d'utiliser un circuit de multiplexage-interpolation fournissant à partir des valeurs des fonctions de corrélation une estimée plus précise de la vitesse et la valeur du pic de corrélation correspondant. La demande de brevet français n° 2 590 790 donne un exemple de ce type de traitement du signal échographique dans lequel la corrélation entre signaux est une corrélation dite "1 bit" en ce sens que les signaux $S_{n+1}$ et $S_n$ utilisés précédemment sont réduits au signe du signal ultrasonore. On sait que dans ce cas, le pic de la fonction de corrélation est de forme triangulaire isocèle. La connaissance de cette forme permet à partir du point le plus haut et de ses deux voisins de reconstituer complètement, par interpolation linéaire, le pic de corrélation et donc de déterminer avec précision l'emplacement de uo.

2

Cette méthode connue de mesure des vitesses, reposant sur l'analyse du décalage temporel, présente de sérieux avantages sur d'autres méthodes fondées, par exemple, sur le décalage de fréquence ou de phase. En particulier, elle permet d'utiliser des signaux d'émission à bande large, produisant une bonne résolution axiale de la mesure.

Toutefois, la méthode exposée ci-dessus ne permet pas de mesurer des vitesses supérieures à une vitesse limite $V_{lim}$ donnée par :

$$V_{lim} = \frac{C}{4} \; \frac{1}{f_o \, T}$$

où C représente la vitesse de propagation de l'onde ultrasonore. Ce phénomène connu aussi sous le terme d'"aliasing" est lié à l'indétermination induite par la périodicité du signal échographique. Une description détaillée en est donnée dans l'ouvrage "Doppler Ultrasound and Its Use in Clinical Measurement", P. Atkinson and J.P. Woodcock, Academic Press, 1982.

A titre d'exemple, avec une période de récurrence T de 100 $\mu$s, une fréquence acoustique centrale $f_o$ de 5 MHz et une vitesse de propagation C de 1500 m/s, on obtient une vitesse limite $V_{lim}$ de 75 cm/s, alors que certains écoulements sanguins, par exemple, peuvent atteindre des vitesses sensiblement supérieures.

Pour augmenter la vitesse limite de mesure, on pourrait penser à diminuer la fréquence $f_o$, mais cela conduirait à une réduction de la précision de la mesure et de la résolution. De même, une augmentation de la fréquence de récurrence aurait pour conséquence indésirable de diminuer la profondeur d'exploration.

Aussi, le problème technique à résoudre par l'objet de la présente invention est de réaliser un dispositif de mesure de la vitesse d'organes en mouvement et d'écoulements sanguins, du type de celui décrit dans le préambule, dispositif qui permettrait d'augmenter la vitesse limite de mesure $V_{lim}$ sans diminuer la fréquence $f_o$ et sans augmenter la fréquence de récurrence $1/T$.

La solution au problème technique posé consiste, selon la présente invention, en ce que ledit dispositif comporte une deuxième voie de traitement du signal échographique reçu, comprenant :
- deux filtres passe-bande symétriques appliqués au signal $S_n(t)$, disposés en parallèle et fournissant respectivement des signaux

$$s_{n_1}(t)$$

et

$$s_{n_2}(t)$$

centrés respectivement autour d'une fréquence $f_1$ au plus égale à $f_o$ et d'une fréquence $f_2$ au moins égale à $f_o$, la différence $f_2-f_1$ étant inférieure à $f_o$,
- un multiplieur réalisant le produit des signaux

$$s_{n_1}(t)$$

et

$$s_{n_2}(t),$$

- un filtre passe-bas symétrique sélectionnant la composante $s_n(t)$ à la fréquence $f_2-f_1$ du produit

$$s_{n_1}(t) \times s_{n_2}(t),$$

- un deuxième circuit d'intercorrélation délivrant $2l+1$ valeurs échantillonnées de la fonction de corrélation de deux signaux $s_n(t)$ et $s_{n+1}(t)$ successifs, dite deuxième fonction de corrélation,

et en ce qu'un circuit de multiplexage-interpolation fournit une estimée de la vitesse par recherche du maximum de la première fonction de corrélation autour de l'échantillon donnant la valeur la plus grande de la deuxième fonction de corrélation.

Ainsi, le dispositif selon l'invention met en jeu non seulement un signal $S_n(t)$ haute fréquence ($f_o$) comme le dispositif connu de l'état de la technique, mais aussi un deuxième signal $s_n(t)$ basse fréquence ($f_2$-$f_1$) qui vérifie également la relation (1) et qui peut donc être traité comme le signal $S_n(t)$.

Dans la mesure où les filtres utilisés pour former le signal basse fréquence sont symétriques et n'introduisent pas de retards ni de phases dans le domaine fréquentiel, et comme le calcul des corrélations se fait dans les mêmes conditions pour l'un et l'autre des deux signaux, les deux fonctions de corrélation, la première et la seconde, coïncident exactement, le maximum de l'une correspondant au maximum de l'autre. Les deux fonctions de corrélation diffèrent en ce que la première, liée au signal $S_n(t)$, a une fréquence plus élevée et présente des pics plus marqués que la seconde, d'où la précision de mesure plus grande, tandis que la seconde fonction de corrélation, liée au signal $s_n(t)$, a une fréquence beaucoup plus faible et ne présente, dans le domaine de mesure considéré, pratiquement qu'un seul maximum. C'est ce qui permet, à l'aide de la deuxième fonction de corrélation, de lever totalement l'indétermination, due au phénomène d'"aliasing", dans la mesure de la vitesse à partir de la première fonction de corrélation.

En conclusion, on constate que le dispositif selon l'invention combine les avantages d'une précision de mesure élevée, déterminée par le signal haute fréquence, et d'une vitesse limite plus grande, imposée par le signal basse fréquence et dont la valeur est donnée par :

$$V_{lim} = \frac{c}{4} \frac{1}{(f_2-f_1)T}$$

On remarquera également qu'indépendamment du phénomène d'"aliasing", le dispositif selon l'invention permet de lever d'autres ambiguïtés, liées à l'échantillonnage, dans la détermination du pic de corrélation. Il peut se produire en effet, que le point le plus haut de la fonction de corrélation échantillonnée n'appartienne pas au pic de corrélation recherché. Cette situation peut survenir lorsqu'on mesure des flux complexes, comportant des gradients de vitesse importants qui tendent à faire baisser le pic de corrélation. Cette erreur se traduit par des discontinuités brutales dans la reconstitution du profil de la vitesse en fonction de la profondeur d'exploration.

La description qui va suivre en regard des dessins annexés, donnés à titre d'exemples non limitatifs, fera bien comprendre en quoi consiste l'invention et comment elle peut être réalisée.

La figure 1 est le schéma d'un dispositif de mesure de vitesse selon l'invention.

La figure 2 donne, de façon schématique, les spectres des signaux impliqués dans le fonctionnement du dispositif de la figure 1.

La figure 3 montre des exemples de fonctions de corrélation obtenues à l'aide du dispositif de la figure 1 et comment elles peuvent être utilisées dans la mesure de la vitesse recherchée.

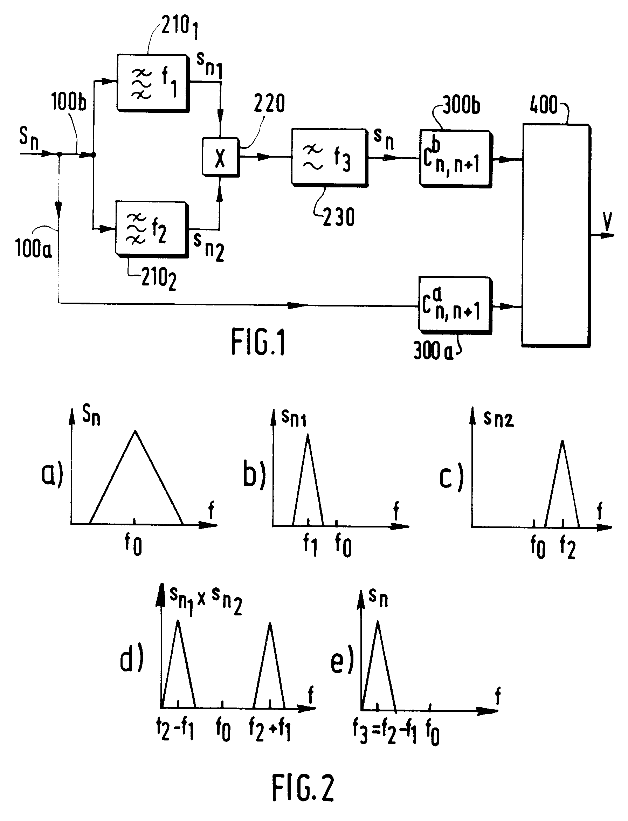

La figure 1 montre, sous forme schématique, un dispositif de mesure de la vitesse d'organes en mouvement et d'écoulements sanguins. Ce dispositif fait partie d'un appareil d'exploration par échographie ultrasonore qui comprend en outre, et non représentés sur la figure 1, au moins un transducteur ultrasonore fournissant une onde acoustique centrée autour d'une fréquence $f_o$ de 5 MHz par exemple, un étage d'émission périodique par ledit transducteur d'un signal impulsionnel de fréquence de récurrence déterminée F = 1/T de 10 kHz par exemple, et un étage de réception des signaux échographiques renvoyés vers le transducteur et de traitement des signaux reçus. La demande de brevet européen n° 0 225 667 donne une description très détaillée de ces différents étages. L'étage de réception et de traitement comporte, notamment, un éliminateur d'échos fixes permettant d'obtenir un signal échographique $S_n(t)$ à la fréquence $f_o$, débarrassée des échos fixes gênants provenant des réflexions sur les parois fixes des organes ou des vaisseaux. Le spectre fréquentiel du signal $S_n(t)$ est très schématiquement représenté sur la figure 2a. Comme le montre la figure 1, le signal $S_n(t)$ est dirigé, sur une première voie 100a de traitement, vers un premier circuit 300a d'intercorrélation comprenant, de façon classique, une ligne à retard d'une période de récurrence T qui permet de recevoir en même temps deux signaux consécutifs $S_n(t)$ et $S_{n+1}(t)$. Puis, 2l + 1 lignes à retard décalent respectivement l'un des deux signaux par rapport à l'autre de la quantité $u_k$ = k$\Delta$t, k étant un nombre entier prenant les valeurs -l, -l + 1, ..., -1, 0, 1, ..., l-1, l et $\Delta$t le pas d'échantillonnage, par exemple de 50 ns. Enfin, des corrélateurs au nombre de 2l + 1 fournissent 2l + 1 valeurs échantillons de la première fonction de corrélation soit :

$$C^a_{n,n+1}(to,u_k) \quad k\varepsilon[-I,I]$$

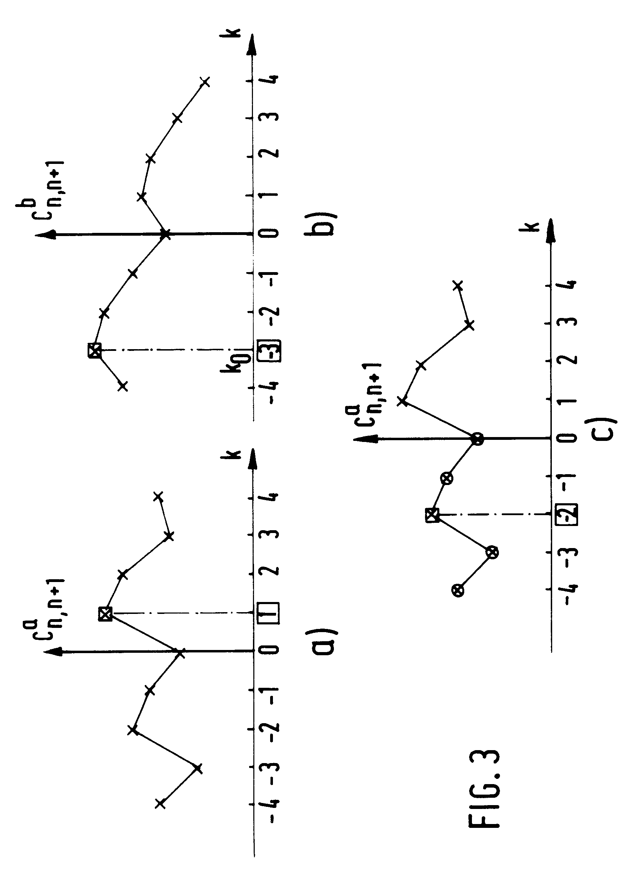

Un exemple d'une telle fonction de corrélation est donné aux figures 3a et 3c, avec I égal à 4.

Le dispositif de la figure comporte également une deuxième voie 100b de traitement du signal échographique reçu, comprenant :

- deux filtres passe-bande $210_1$, $210_2$ symétriques appliqués au signal $S_n(t)$, disposés en parallèle et qui fournissent respectivement des signaux

$$s_{n_1}(t)$$

et

$$s_{n_2}(t)$$

centrés respectivement autour d'une fréquence $f_1$ au plus égale à fo, par exemple 4 MHz, et d'une fréquence $f_2$ au moins égale à $f_o$, par exemple 6 MHz, la différence $f_2 - f_1 = 2$ MHz étant inférieure à $f_o$ = 5 MHz. Bien entendu, les fréquences $f_1$ et $f_2$ doivent, comme l'indiquent les figures 2b et 2c se situer dans la bande de fréquence ultrasonore centrée autour de $f_o$.

Par filtres symétriques, on entend des filtres dont la réponse temporelle h(t) vérifie h(-t) = h(t). D'autre part, les signaux

$$s_{n_1}(t)$$

et

$$s_{n_2}(t)$$

obéissent à la relation générale (1) rappelée plus haut.
- un multiplieur 220 qui réalise le produit des signaux

$$s_{n_1}(t)$$

et

$$s_{n_2}(t),$$

ce qui fait apparaître sur le spectre de fréquence (figure 2d) deux signaux, l'un basse fréquence centré sur $f_2 - f_1$, et l'autre haute fréquence centré sur $f_2 + f_1$.
- un filtre passe-bas (230) également symétrique sélectionnant la composante $s_n(t)$ à la fréquence $f_3$ = $f_2 - f_1$ du produit

$$s_{n_1}(t) \times s_{n_2}(t),$$

ainsi qu'on peut le voir à la figure 2e.
- un deuxième circuit 300b d'intercorrélation, analogue à celui 300a de la première voie 100a de traitement, qui délivre 2I+1 valeurs échantillonnées, au pas d'échantillonnage $\Delta$t, de la fonction de corrélation

$$c_{n,n+1}^{b}(to,u_k)$$

de deux signaux $s_n(t)$ et $s_{n+1}(t)$ successifs. La figure 3b montre un exemple de deuxième fonction de corrélation

$$c_{n,n+1}^{b}(to,u_k)$$

qui, comme on peut le voir, a une fréquence plus basse que la première fonction de corrélation

$$c_{n,n+1}^{a}.$$

Les corrélateurs utilisés dans l'un et l'autre des circuits 300a et 300b d'intercorrélation peuvent être des corrélateur dits "1 bit", comme rappelé plus haut, qui donnent aux fonctions de corrélation de la figure 3 une forme triangulaire.

En sortie des circuits d'intercorrélation, un circuit de multiplexage-interpolation fournit une estimée de la vitesse V par recherche du maximum de la première fonction de corrélation

$$c_{n,n+1}^{a}$$

autour de l'échantillon ko donnant la valeur la plus grande de la deuxième fonction de corrélation

$$c_{n,n+1}^{b}.$$

La figure 3 illustre cette démarche sur un exemple concret. La première fonction de corrélation

$$c_{n,n+1}^{a}$$

présente deux valeurs maximales relatives pour les échantillons k = -2 et k = 1 et, a priori, la valeur maximale à k = 1 étant la plus grande, on pourrait penser que la vitesse V est voisine de $kC\Delta t/2T$ soit $C\Delta t/2T$. En fait, la deuxième courbe de corrélation

$$c_{n,n+1}^{b}$$

lève cette indétermination car elle indique clairement que le calcul de la vitesse doit se faire autour de l'échantillon ko = -3, ce qui impose l'échantillon k = -2, deuxième valeur maximale de

$$c_{n,n+1}^{a}.$$

L'interpolation linéaire destinée à déterminer avec plus de précision la position du maximum de

$$c_{n,n+1}^{a}$$

autour de k = -2 se fait avec 5 échantillons encadrant k = -2 soit -4, -3, -2, -1, 0. Ce nombre 5 de points choisis provient du fait que dans l'exemple considéré ici, la fréquence d'échantillonnage $1/\Delta T$ est 4 fois la fréquence $f_o$ ; on peut donc faire 5 échantillons dans une période de signal ultrasonore. Cette interpolation linéaire, liée à la corrélation 1 bit reconstruit le triangle isocèle à partir du point maximum et des deux points adjacents. Avec les valeurs numériques précédentes pour $f_o$, $f_1$ et $f_2$, on voit que la valeur limite $V_{lim}$ de la vitesse est multipliée par $f_o/(f_2-f_1)$ soit 2,5. On peut ainsi atteindre des vitesses près de 200 cm/s, soit toutes les vitesses de mouvements ou d'écoulements du corps humain.

**Revendications**

**1.** Dispositif de mesure de la vitesse d'organes en mouvement et d'écoulements sanguins par échographie ultrasonore utilisant une onde acoustique de fréquence centrale $f_o$, et comportant une première voie (100a) de traitement du signal échographique reçu, comprenant un premier circuit (300a) d'intercorrélation délivrant $2l+1$ valeurs échantillonnées de la fonction de corrélation de deux signaux échographiques $S_n(t)$ et $S_{n+1}(t)$ successifs, dite première fonction de corrélation, caractérisé en ce que ledit dispositif comporte une deuxième voie (100b) de traitement du signal échographique reçu, comprenant :

- deux filtres passe-bande ($210_1$, $210_2$) symétriques appliqués au signal $S_n(t)$, disposés en parallèle et fournissant respectivement des signaux

$$s_{n_1}(t)$$

et

$$s_{n_2}(t)$$

centrés respectivement autour d'une fréquence $f_1$ au plus égale à $f_o$ et d'une fréquence $f_2$ au moins égale à $f_o$, la différence $f_2-f_1$ étant inférieure à $f_o$,
- un multiplieur (220) réalisant le produit des signaux

$$s_{n_1}(t)$$

et

$$s_{n_2}(t),$$

- un filtre passe-bas (230) symétrique sélectionnant la composante $s_n(t)$ à la fréquence $f_2-f_1$ du produit

$$s_{n_1}(t) \times s_{n_2}(t),$$

- un deuxième circuit (300b) d'intercorrélation délivrant $2l+1$ valeurs échantillonnées de la fonction de corrélation de deux signaux $s_n(t)$ et $s_{n+1}(t)$ successifs, dite deuxième fonction de corrélation,

et en ce qu'un circuit (400) de multiplexage-interpolation fournit une estimée de la vitesse par recherche des coordonnées du maximum de la première fonction de corrélation autour de l'échantillon (ko) donnant la valeur la plus grande de la deuxième fonction de corrélation.

**2.** Dispositif selon la revendication 1, caractérisé en ce que lesdits circuits (300a,300b) d'intercorrélation comportent des corrélateurs 1 bit, et en ce que ledit circuit (400) de multiplexage-interpolation effectue une interpolation reconstruisant le triangle isocèle.

**Claims**

**1.** A device for measuring of the speed of moving organs and blood flows by ultrasonic echography, using an acoustic wave having a central frequency $f_o$, which device comprises a first processing channel (100a) for the echographic signal received, which channel comprises a first correlation circuit (300a) which supplies $2l+1$ sampled values of the correlation function of two successive echographic signals

$S_n(t)$ and $S_{n+1}(t)$, referred to as the first correlation function, characterized in that said device comprises a second processing channel (100b) for the echographic signal received, comprising

- two symmetrical bandpass filters ($210_1$, $210_2$) which act on the signal $S_n(t)$, are connected in parallel, and supply signals $s_{n1}(t)$ and $s_{n2}(t)$, respectively, which are centred around a frequency $f_1$, being at the most equal to $f_o$, and around a frequency $f_2$, being at least equal to $f_o$, respectively, the difference $f_2$-$f_1$ being smaller than $f_o$,
- a multiplier (220) which forms the product of the signals $s_{n1}(t)$ and $s_{n2}(t)$,
- a symmetrical bandpass filter (230) which selects the component $s_n(t)$ having the frequency $f_2$-$f_1$ of the product $s_{n1}(t)$ x $s_{n2}(t)$,
- a second correlation circuit (300b) which supplies $2I+1$ sampled values of the correlation function of two successive signals $s_n(t)$ and $s_{n+1}(t)$, referred to as the second correlation function,

and in that a multiplexing/interpolation circuit (400) supplies an estimate of the speed by searching the coordinates of the maximum of the first correlation function around the sample (ko) offering the highest value of the second correlation function.

2.  A device as claimed in Claim 1, characterized in that said correlation circuits (300a, 300b) comprise 1-bit correlators, and in that said multiplexing/interpolation circuit (400) performs an interpolation reconstructing the isosceles triangle.


**Patentansprüche**

1.  Vorrichtung zur Messung der Geschwindigkeit von Organbewegungen und Blutströmungen mit Hilfe der Ultraschallechographie unter Verwendung einer akustischen Welle mit einer Mittenfrequenz $f_o$, mit einem ersten Kanal (100a) zur Verarbeitung des erhaltenen Echographiesignals, der eine erste Interkorrelationsschaltung (300a) umfaßt, die $2I+1$ Abtastwerte der Korrelationsfunktion von zwei aufeinanderfolgenden Echographiesignalen $S_n(t)$ und $S_{n+1}(t)$ liefert, die als erste Korrelationsfunktion bezeichnet wird, dadurch gekennzeichnet, daß die Vorrichtung einen zweiten Kanal (100b) zur Verarbeitung des erhaltenen Echographiesignals umfaßt, mit:
    - zwei parallel geschalteten symmetrischen Bandpaßfiltern ($210_1$, $210_2$) für das Signal $S_n(t)$, die Signale $s_{n1}(t)$ bzw. $s_{n2}(t)$ liefern, die jeweils um eine Frequenz $f_1$, die höchstens gleich $f_o$ ist, bzw. eine Frequenz $f_2$, die höchstens gleich $f_o$ ist, zentriert sind, wobei die Differenz $f_2$-$f_1$ kleiner als $f_o$ ist,
    - einem Multiplizierer (220) zum Bilden des Produkts der Signale $s_{n1}(t)$ und $s_{n2}(t)$,
    - einem symmetrischen Bandpaßfilter (230), das die Komponente $s_n(t)$ bei der Frequenz $f_2$-$f_1$ des Produkts $s_{n1}(t)$ x $s_{n2}(t)$ selektiert,
    - einer zweiten Interkorrelationsschaltung (300b), die $2I+1$ Abtastwerte der Korrelationsfunktion von zwei aufeinanderfolgenden Echographiesignalen $s_n(t)$ und $s_{n+1}(t)$ liefert, die als zweite Korrelationsfunktion bezeichnet wird,

    und daß eine Multiplexinterpolationsschaltung (400) durch Aufsuchen der Koordinaten des Maximums der ersten Korrelationsfunktion um den Abtastwert (ko) herum, der den größten Wert der zweiten Korrelationsfunktion ergibt, eine Schätzung der Geschwindigkeit liefert.

2.  Vorrichtung nach Anspruch 1, dadurch gekennzeichnet, daß die genannten Interkorrelationsschaltungen (300a, 300b) 1-Bit-Korrelatoren umfassen und daß die genannte Multiplexinterpolationsschaltung (400) eine Interpolation vornimmt, die das gleichschenklige Dreieck rekonstruiert.

FIG.1

FIG.2

FIG. 3